(19) Europäisches Patentamt
European Patent Office
Office européen des brevets

(11) EP 3 520 674 A1

(12) EUROPEAN PATENT APPLICATION
published in accordance with Art. 153(4) EPC

(43) Date of publication:
07.08.2019 Bulletin 2019/32

(51) Int Cl.:
A61B 1/005 (2006.01)    G02B 23/24 (2006.01)

(21) Application number: 17856279.9

(22) Date of filing: 27.09.2017

(86) International application number:
PCT/JP2017/035077

(87) International publication number:
WO 2018/062330 (05.04.2018 Gazette 2018/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 28.09.2016  JP 2016189760

(71) Applicant: Fujifilm Corporation
Minato-ku
Tokyo 106-8620 (JP)

(72) Inventor: NAKAI, Yoshihiro
Ashigarakami-gun
Kanagawa 258-8577 (JP)

(74) Representative: Hoffmann Eitle
Patent- und Rechtsanwälte PartmbB
Arabellastraße 30
81925 München (DE)

(54) ENDOSCOPE FLEXIBLE TUBE SHEET, ENDOSCOPE FLEXIBLE TUBE, ENDOSCOPE, AND ENDOSCOPE FLEXIBLE TUBE SHEET PRODUCTION METHOD

(57) Provided are a sheet for an endoscope flexible tube which is formed of a composite material that contains a fluorine-containing resin component and a fluorine-containing rubber component and is obtained by polyol-crosslinking of at least parts of the fluorine-containing resin component and the fluorine-containing rubber component; an endoscope flexible tube; an endoscope; and a method of producing a sheet for an endoscope flexible tube.

FIG. 2

**Description**

**BACKGROUND OF THE INVENTION**

1. Field of the Invention

The present invention relates to a sheet for an endoscope flexible tube, an endoscope flexible tube, an endoscope, and a method of producing a sheet for an endoscope flexible tube.

2. Description of the Related Art

An endoscope is medical equipment for observing the inside of the body cavity of a human body. Since an insertion portion of an endoscope is used by being inserted into the body cavity, it is desired that the endoscope does not damage organs and does not give pain or discomfort to a subject. Due to such a request, a helix tube formed by spirally winding a metal strip which is softly bent is employed as a base material for a flexible tube constituting an insertion portion of an endoscope. Further, the surroundings thereof are covered with a flexible resin, and a method has been devised not to stimulate or damage the surface of the esophagus or intestines.

An endoscope is repeatedly used. Therefore, it is necessary that a flexible tube constituting an insertion portion of an endoscope is washed every time it is used and disinfected using chemicals. For the purpose of preventing infections, in a case where an endoscope is inserted to a site such as the bronchus with a high possibility of infections, since high cleanliness in a level of sterilization rather than disinfection is required, application of a sterilization treatment using an autoclave as well as a sterilization treatment using ethylene oxide gas (EOG) which has been widely performed are also desired recently. In order to realize an endoscope capable of withstanding such a high degree of sterilization treatment, further improvement of the chemical resistance, the heat resistance, and the like of an endoscope has been examined (for example, see JP2006-081663A).

**SUMMARY OF THE INVENTION**

In a flexible tube of an endoscope, a resin covering a base material is required to have excellent mechanical characteristics (the hardness, tensile strength, tear strength, or the like) and also required to have characteristics (high chemical resistance and heat resistance) in which the above-described characteristics can be sufficiently maintained even in a case where a strong sterilization treatment is performed.

JP2006-081663A describes an elastomer molded body for an endoscope which is obtained by causing a crosslinking reaction in a mixture containing a perfluoroelastomer and a filler, as a resin for covering a flexible tube base material, at a specific amount ratio using a crosslinking agent and a co-crosslinking agent, and molding the resulting mixture. JP2006-081663A also describes that this elastomer molded body for an endoscope has excellent resistance particularly to a high-pressure steam sterilization treatment carried out using an autoclave. However, it is difficult to realize a sufficient level of hardness required for an endoscope flexible tube in a case where the elastomer molded body described in JP2006-081663A is used. Further, it has been thought that cracks or fractures are unlikely to occur in the elastomer molded body described in JP2006-081663A even at the time of autoclave sterilization. However, the elastomer molded body is not capable of sufficiently suppress plastic deformation and is solidified (having a so-called bending habit) in a shape of being stored in a narrow autoclave furnace in a state in which the flexible tube is bent at the time of autoclave sterilization. Therefore, there is a concern that an insertion operation of the endoscope flexible tube which is substantially carried out is disturbed in a case where the elastomer molded body is used.

The present invention has been made in consideration of the above-described circumstances, and an object thereof is to provide a sheet for an endoscope flexible tube which is used as a covering material that covers a base material in an endoscope flexible tube, has excellent hardness, tensile strength, and tear strength and excellent chemical resistance to chemicals used for disinfection or EOG sterilization, and has high heat resistance so that the sheet is unlikely to have a bending habit even in a case where autoclave sterilization is repeatedly performed under a high temperature condition (for example, in a temperature region of 120° to 135°C). Further, another object of the present invention is to provide an endoscope flexible tube and an endoscope formed of this sheet for an endoscope flexible tube. Further, a still another object of the present invention is to provide a method of producing a sheet for an endoscope flexible tube having the above-described characteristics.

As the result of intensive examination conducted by the present inventors, it was found that a sheet satisfying the above-described characteristics is obtained by combining a fluorine-containing resin component and a fluorine-containing rubber component as a resin component constituting a sheet for an endoscope flexible tube and setting a state in which at least a part of the fluorine-containing resin component and at least a part of the fluorine-containing rubber component are polyol-crosslinked. The present invention has been completed based on these findings.

The above-described problems are solved by the following means.

<1> A sheet for an endoscope flexible tube which is formed of a composite material that contains a fluorine-containing resin component and a fluorine-containing rubber component and is obtained by polyol-crosslinking of at least parts of the fluorine-containing resin component and the fluorine-containing rubber component.

<2> The sheet for an endoscope flexible tube according to <1>, in which the fluorine-containing resin component contains at least one fluorine-containing resin component selected from polyvinylidene fluoride, an ethylene-tetrafluoroethylene copolymer, an ethylene-tetrafluoroethylene-hexafluoropropylene terpolymer, a tetrafluoroethylene-hexafluoropropylene copolymer, polychlorotrifluoroethylene, or a modified perfluoroalkoxy resin.

<3> The sheet for an endoscope flexible tube according to <1>, in which the fluorine-containing rubber component contains at least one fluorine-containing rubber component selected from a vinylidene fluoride-hexafluoropropylene copolymer, a vinylidene fluoride-hexafluoropropylene-tetrafluoroethylene terpolymer, tetrafluoroethylene-propylene rubber, a vinylidene fluoride-pentafluoropropylene copolymer, a vinylidene fluoride-chlorotrifluoropropylene copolymer, or tetrafluoroethylene-perfluoromethyl vinyl ether rubber.

<4> The sheet for an endoscope flexible tube according to any one of <1> to <3>, in which the composite material contains a copolymer component having a partial structure formed of one fluorine-containing resin component and a partial structure formed of one fluorine-containing rubber component.

<5> An endoscope flexible tube comprising: the sheet for an endoscope flexible tube according to any one of <1> to <4>, as an outer skin.

<6> An endoscope comprising: the endoscope flexible tube according to <5>.

<7> A method of producing the sheet for an endoscope flexible tube according to any one of <1> to <4>, comprising: a step of mixing a fluorine-containing resin component and a fluorine-containing rubber component; and a step of causing at least parts of the mixed fluorine-containing resin component and fluorine-containing rubber component to be polyol-crosslinked.

In the present specification, the "fluorine-containing resin component" indicates a component which contains a fluorine atom and whose bending elastic modulus at 23°C is 20 MPa or greater. Further, the "fluorine-containing rubber component" indicates a component which contains a fluorine atom and whose bending elastic modulus at 23°C is less than 20 MPa. The fluorine-containing resin component may be present in the form of a single fluorine-containing resin component (in other words, present independently without being crosslinked or the like) or may be present in a state in which the fluorine-containing resin component and another fluorine-containing resin component or the fluorine-containing resin component and another fluorine-containing rubber component are polyol-crosslinked, in the sheet for an endoscope flexible tube of the present invention. The same applies to the fluorine-containing rubber component. Further, these components may be present in the form of a copolymer having a fluorine-containing resin component and a fluorine-containing rubber component as partial structures. Here, in the sheet for an endoscope flexible tube of the present invention, at least a part of the fluorine-containing resin component and at least a part of the fluorine-containing rubber component are present in a state of being polyol-crosslinked.

Further, the numerical values shown using "to" in the present specification indicate that the numerical values described before and after "to" are included as the lower limits and upper limits.

Further, the mass average molecular weight in the present specification is a value measured by gel permeation chromatography (GPC) (in terms of polystyrene) unless otherwise specified.

The sheet for an endoscope flexible tube of the present invention has excellent hardness, tensile strength, and tear strength, excellent chemical resistance, and high heat resistance so that the sheet is unlikely to have a bending habit even in a case where autoclave sterilization is repeatedly performed under a high temperature condition (for example, in a temperature region of 120° to 135°C). Further, according to the present invention, it is possible to provide an endoscope flexible tube and an endoscope formed of a sheet for an endoscope flexible tube exerting the above-described excellent effects. Further, according to the method of producing a sheet for an endoscope flexible tube of the present invention, it is possible to provide a sheet for an endoscope flexible tube having the above-described characteristics.

The above-described features, other features, and advantages of the present invention will become more apparent based on the following description with reference to the accompanying drawings as appropriate.

**BRIEF DESCRIPTION OF THE DRAWINGS**

Fig. 1 is an external view illustrating a configuration of an example of an endoscope according to the present invention.
Fig. 2 is a partial cross-sectional view illustrating a schematic configuration of an example of an endoscope flexible tube according to the present invention, which constitutes the endoscope illustrated in Fig. 1.

**DESCRIPTION OF THE PREFERRED EMBODIMENTS**

<Sheet for endoscope flexible tube>

A sheet for an endoscope flexible tube of the present invention is formed of a composite material that contains a fluorine-containing resin component and a fluorine-containing rubber component. In this composite material, at least parts of the fluorine-containing resin component and the fluorine-containing rubber component are in a state of being polyol-crosslinked.

(Fluorine-containing resin component)

The fluorine-containing resin component used in the composite material is not particularly limited as long as the component is a compound satisfying the above-described definition, and the component formed of a polymer obtained by polymerizing one or two or more of vinyl fluoride, vinylidene fluoride, ethylene, tetrafluoroethylene, propylene, chlorotrifluoropropylene, hexafluoropropylene, chlorotrifluoroethylene, trifluoroethylene, hexafluoroisobutene, perfluoro(methylvinylether), perfluoro(propylvinylether), and perfluoroalkoxyethylene is preferable. Specific examples of this polymer include polyvinyl fluoride, polyvinylidene fluoride, polytetrafluoroethylene, an ethylene-tetrafluoroethylene copolymer, an ethylene-tetrafluoroethylene-hexafluoropropylene terpolymer, a tetrafluoroethylene-hexafluoropropylene copolymer, polychlorotrifluoroethylene, an ethylene-chlorotrifluoroethylene copolymer, a vinylidene fluoride-chlorotrifluoroethylene copolymer, a vinylidene fluoride-chlorotrifluoroethylene-tetrafluoroethylene terpolymer, and a tetrafluoroethylene-perfluoroalkoxyethylene copolymer. Further, in the present invention, it is also preferable that a modified perfluoroalkoxy resin is used. The modified perfluoroalkoxy resin indicates a resin obtained by allowing a copolymer of tetrafluoroethylene and chlorotrifluoroethylene to contain a modified group.

In the present invention, it is preferable that at least one selected from polyvinylidene fluoride, an ethylene-tetrafluoroethylene copolymer, an ethylene-tetrafluoroethylene-hexafluoropropylene terpolymer, a tetrafluoroethylene-hexafluoropropylene copolymer, polychlorotrifluoroethylene, a modified perfluoroalkoxy resin, and a tetrafluoroethylene-perfluoroalkoxyethylene copolymer is used.

Further, since the hardness of the sheet for an endoscope flexible tube is increased, it is more preferable that at least one selected from polyvinylidene fluoride, an ethylene-tetrafluoroethylene copolymer, a tetrafluoroethylene-hexafluoropropylene copolymer, and a tetrafluoroethylene-perfluoroalkoxyethylene copolymer is used.

The fluorine-containing resin component may be used alone or in combination of two or more kinds thereof.

In a case where the fluorine-containing resin component is a component formed of a copolymer, the component may be present in the form of any of a statistic copolymer, an alternating copolymer, a periodic copolymer, a block copolymer, and a graft copolymer.

The melt flow rate which is an indicator of the molecular weight of the fluorine-containing resin component is not particularly limited, but is preferably in a range of 1 to 120 g/10 min and more preferably in a range of 3 to 80 g/10 min. In a case where the melt flow rate of the fluorine-containing resin component is in the above-described range, the moldability is excellent, and the tensile strength and the tear strength are increased. Further, the melt flow rate is acquired according to the measuring method described in the section of examples below.

The content of the fluorine-containing resin component in the composite material is preferably in a range of 10% to 99% by mass and more preferably in a range of 30% to 90% by mass.

The composite material may contain one fluorine-containing resin component or in combination of two or more fluorine-containing resin components. The fluorine-containing resin component can be synthesized according to a method of the related art or commercially available products can be used as the fluorine-containing resin component.

(Fluorine-containing rubber component)

The fluorine-containing rubber component used in the composite material is not particularly limited as long as the component is a compound satisfying the above-described definition, and a compound formed of a polymer obtained by polymerizing one or two or more of vinylidene fluoride, tetrafluoroethylene, hexafluoropropylene, vinyl fluoride, chlorotrifluoroethylene, trifluoroethylene, trifluoropropylene, tetrafluoropropylene, pentafluoropropylene, trifluorobutene, tetrafluoroisobutene, and perfluoro(alkylvinylether) is preferable. Specific examples of this polymer include a vinylidene fluoride-hexafluoropropylene copolymer, a vinylidene fluoride-hexafluoropropylene-tetrafluoroethylene terpolymer, tetrafluoroethylene-propylene rubber, a vinylidene fluoride-pentafluoropropylene copolymer, a vinylidene fluoride-chlorotrifluoropropylene copolymer, tetrafluoroethylene-perfluoromethylvinylether rubber, a vinylidene fluoride-perfluoro(alkylvinylether) copolymer, a vinylidene fluoride-tetrafluoroethylene-perfluoro(alkylvinylether) terpolymer, a vinylidene fluoride-hexafluoropropylene-perfluoro(alkylvinylether) terpolymer, and a vinylidene fluoride-tetrafluoroethylene-hexafluoropropylene-perfluoro(alkylvinylether) tetrapolymer.

In the present invention, it is preferable that at least one selected from a vinylidene fluoride-hexafluoropropylene copolymer, vinylidene fluoride-hexafluoropropylene-tetrafluoroethylene terpolymer, tetrafluoroethylene-propylene rubber, a vinylidene fluoride-pentafluoropropylene copolymer, a vinylidene fluoride-chlorotrifluoropropylene copolymer, and tetrafluoroethylene-perfluoromethylvinylether rubber is used.

Among these, from the viewpoint that the hardness and the heating residual stress of the sheet for an endoscope flexible tube can be increased, it is preferable that at least one selected from a vinylidene fluoride-hexafluoropropylene copolymer, and a vinylidene fluoride-hexafluoropropylene-tetrafluoroethylene terpolymer is used.

The fluorine-containing rubber component may be used alone or in combination of two or more kinds thereof.

In a case where the fluorine-containing rubber component is a component formed of a copolymer, the component may be present in the form of any of a statistic copolymer, an alternating copolymer, a periodic copolymer, a block copolymer, and a graft copolymer.

The mass average molecular weight of the fluorine-containing rubber component is not particularly limited, but is preferably in a range of 20000 to 2000000 and more preferably in a range of 50000 to 500000. The mass average molecular weight of the fluorine-containing rubber component is in the above-described range, the molding processability becomes excellent, and the tensile strength and the tear strength are increased. The mass average molecular weight can be measured by preparing a GPC device HLC-8220 (trade name, manufactured by Tosoh Corporation) using an RI detector under a temperature condition of 23°C at a flow rate of 1 mL/min using tetrahydrofuran (manufactured by Shonan Wako Junyaku K.K.) as an eluent, and TSK gel (registered trademark) G3000HXL and TSK gel (registered trademark) G2000HXL (both trade names) as columns.

The content of the fluorine-containing rubber component in the composite material is preferably in a range of 1% to 90% by mass and more preferably in a range of 10% to 70% by mass.

The composite material may contain one fluorine-containing rubber component or in combination of two or more fluorine-containing rubber components. The fluorine-containing rubber component can be synthesized according to a method of the related art or commercially available products can be used as the fluorine-containing rubber component.

The ratio between the content of the fluorine-containing resin component and the content of the fluorine-containing rubber component in the composite material is not particularly limited, but is preferably in a range of 10:90 to 99:1 and more preferably in a range of 30: 70 to 90:10. In a case where the ratio between the content of the fluorine-containing resin component and the content of the fluorine-containing rubber component is in the above-described range, the hardness of the sheet for an endoscope flexible tube is high and the heating residual stress is increased.

(Copolymer component)

It is preferable that the composite material contains a copolymer component having a partial structure formed of one fluorine-containing resin component and a partial structure formed of one fluorine-containing rubber component. In a case where the composite material contains such a copolymer component, the overall compatibility between the fluorine-containing resin component and the fluorine-containing rubber component constituting the sheet for an endoscope flexible tube is improved. As the result, the hardness, the tensile strength, the tear strength, and the heat resistance of the sheet for an endoscope flexible tube can be further improved so that the bending habit can be more effectively suppressed.

In the sheet for an endoscope flexible tube of the present invention, from the viewpoint of efficiently suppressing the bending habit, it is preferable that 1% to 50% by mass of the entire fluorine-containing resin component present in the composite material constituting the sheet for an endoscope flexible tube is present as a copolymer with the fluorine-containing rubber component and more preferable that 3% to 40% by mass thereof is present as a copolymer with the fluorine-containing rubber component.

(Polyol crosslinking)

As described above, in the sheet for an endoscope flexible tube of the present invention, at least a part of the fluorine-containing resin component and the fluorine-containing rubber component in the composite material is present in a state of being polyol-crosslinked. In other words, a form in which at least two compounds constituting the fluorine-containing resin component are crosslinked and the crosslinking point has a carbon-oxygen bond, a form in which at least two compounds constituting the fluorine-containing rubber component are crosslinked and the crosslinking point has a carbon-oxygen bond, and/or a form in which at least one compound constituting the fluorine-containing resin component and at least one compound constituting the fluorine-containing rubber component are crosslinked and the crosslinking point has a carbon-oxygen bond may be exemplified.

Further, the "carbon" in a carbon-oxygen bond indicates a carbon atom constituting the fluorine-containing resin component and/or the fluorine-containing rubber component. Further, the "oxygen" in the carbon-oxygen bond indicates an oxygen atom constituting a component derived from a polyol-crosslinking agent described below.

The sheet for an endoscope flexible tube of the present invention has excellent hardness, tensile strength, and tear strength because the sheet contains a fluorine-containing resin component whose bending elastic modulus at 23° is 20 MPa or greater and a fluorine-containing rubber component whose bending elastic modulus at 23°C is less than 20 MPa. Further, the bending habit can be effectively suppressed by the polyol-crosslinking even in a case where the sheet is exposed to a high temperature such as autoclave sterilization. The reason for this is not clear, but it is considered that at least one of the fluorine-containing resin component or the fluorine-containing rubber component forms a crosslinked structure so that the initial shape is held, and thus plastic deformation is suppressed.

Further, the bending elastic modulus of the fluorine-containing resin component at 23°C is preferably 100 MPa or greater and the upper limit thereof is practically 5 GPa or less. The bending elastic modulus of the fluorine-containing rubber component at 23°C is preferably 10 MPa or greater and the lower limit thereof is practically 0.1 MPa or greater. The bending elastic modulus is acquired by the measuring method described in the section of examples below.

(Crosslinking agent)

In the sheet for an endoscope flexible tube of the present invention, at least a part of the fluorine-containing resin component and/or the fluorine-containing rubber component is polyol-crosslinked through a polyol-crosslinking agent.

The polyol-crosslinking agent used for the sheet for an endoscope flexible tube of the present invention is not particularly limited. For example, a polyhydroxy compound is preferable. Among the examples of the polyhydroxy compound, a polyhydroxy aromatic compound is preferably used. Specific examples of the polyhydroxy aromatic compound include 2,2-bis(4-hydroxyphenyl)propane (bisphenol A), 2,2-bis(4-hydroxyphenyl)perfluoropropane (bisphenyl AF), resorcin, 1,3-dihydroxybenzene, 1,7-dihydroxynaphthalene, 2,7-dihydroxynaphthalene, 1,6-dihydroxynaphthalene, 4,4'-dihydroxydiphenyl, 4,4'-dihydroxystilbene, 2,6-dihydroxyanthracene, hydroquinone, catechol, 2,2-bis(4-hydroxyphenyl)butane (bisphenol B), 4,4-bis(4-hydroxyphenyl)valeric acid, 2,2-bis(4-hydroxyphenyl)tetrafluorodichloropropane, 4,4'-dihydroxydiphenylsulfone, 4,4'-dihydroxydiphenylketone, tri(4-hydroxyphenyl)methane, and 3,3',5,5'-tetrachlorobisphenol A, 3,3',5,5'-tetrabromobisphenol A.

In the sheet for an endoscope flexible tube of the present invention, the content ratio of the component derived from a polyol-crosslinking agent in the composite material is preferably in a range of 0.01% to 15% by mass and more preferably in a range of 0.1% to 8% by mass. Further, the component derived from a polyol-crosslinking agent indicates a component formed of an atomic group from an oxygen atom of a carbon-oxygen bond at one crosslinking point to an oxygen atom at another crosslinking point.

The fluorine-containing rubber component may be used alone or in combination of two or more kinds thereof. The fluorine-containing rubber component can be synthesized according to a method of the related art or commercially available products can be used as the fluorine-containing rubber component.

(Crosslinking promoter)

The sheet for an endoscope flexible tube of the present invention may contain a component derived from a crosslinking promoter used at the time of causing polyol-crosslinking described above. Specific examples of the crosslinking promoter include an ammonium compound such as calcium hydroxide, magnesium oxide, or a quaternary ammonium salt, a phosphonium compound such as a quaternary phosphonium salt, an oxonium compound, a sulfonium compound, a cyclic amine, and a monofunctional amine compound.

The quaternary ammonium salt is not particularly limited, and examples thereof include 8-methyl-1,8-diazabicyclo[5.4.0]-7-undecenium chloride, 8-methyl-1,8-diazabicyclo[5.4.0]-7-undecenium iodide, 8-methyl-1,8-diazabicyclo[5.4.0]-7-undecenium hydroxide, 8-methyl-1,8-diazabicyclo[5.4.0]-7-undecenium methyl sulfate, 8-ethyl-1,8-diazabicyclo[5.4.0]-7-undecenium bromide, 8-propyl-1,8-diazabicyclo[5.4.0]-7-undecenium bromide, 8-dodecyl-1,8-diazabicyclo[5.4.0]-7-undecenium chloride, 8-dodecyl-1,8-diazabicyclo[5.4.0]-7-undecenium hydroxide, 8-eicosyl-1,8-diazabicyclo[5.4.0]-7-undecenium chloride, 8-tetracosyl-1,8-diazabicyclo [5.4.0]-7-undecenium chloride, 8-benzyl-1,8-diazabicyclo[5.4.0]-7-undecenium chloride (hereinafter, referred to as DBU-B), 8-benzyl-1,8-diazabicyclo[5.4.0]-7-undecenium hydroxide, 8-phenethyl-1,8-diazabicyclo[5.4.0]-7-undecenium chloride, and 8-(3-phenylpropyl)-1,8-diazabicyclo[5.4.0]-7-undecenium chloride.

Further, as the quaternary phosphonium salt, for example, tetrabutyl phosphonium chloride, benzyl triphenyl phosphonium chloride (hereinafter, referred to as BTPPC), benzyl trimethyl phosphonium chloride, benzyl tributyl phosphonium chloride, tributyl allyl phosphonium chloride, tributyl-2-methoxypropyl phosphonium chloride, or benzyl phenyl (dimethylamino)phosphonium chloride is preferable.

(Other components)

The sheet for an endoscope flexible tube of the present invention may contain, within the range exhibiting the effects of the present invention, various additives such as a filler, a processing aid, a plasticizer, a colorant, a stabilizer, an adhesion assistant, a releasing agent, a conductivity imparting agent, a thermal conductivity imparting agent, a surface non-pressure sensitive adhesive, a flexibility imparting agent, a heat resistant improving agent, and a flame retardant.

<Endoscope>

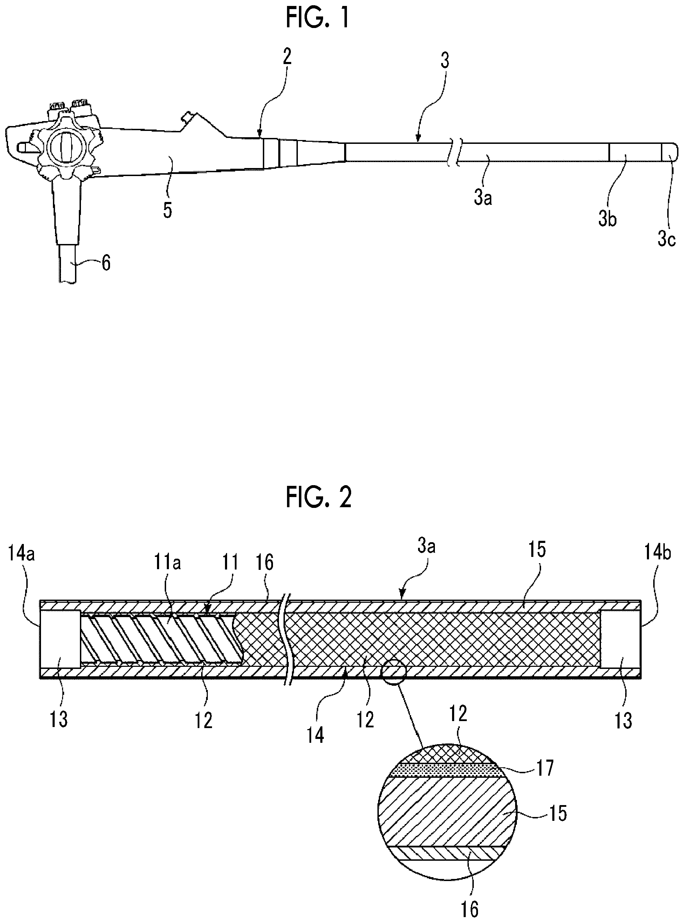

An endoscope flexible tube 3a of the present invention (hereinafter, referred to as a flexible tube 3a) is incorporated in an electronic endoscope 2 which is an example of the endoscope according to the present invention, illustrated in Fig. 1. Such products are widely used for medical use. In the example illustrated in Fig. 1, the electronic endoscope 2 includes an insertion portion 3 to be inserted into the body cavity, a main body operation unit 5 consecutively connected to a proximal end portion of the insertion portion 3, and a universal cord 6 to be connected to a processor device or a light source device. The insertion portion 3 is formed of the flexible tube 3a consecutively connected to the main body operation unit 5, an angle portion 3b consecutively connected thereto, and a distal end portion 3c which is consecutively connected to the distal end thereof and in which an imaging device (not illustrated) for imaging the inside of the body cavity is built in. The flexible tube 3a having a length which occupies the most of the length of the insertion portion 3 has a flexibility substantially over the entire length thereof, and particularly a portion to be inserted into the body cavity or the like is more flexible than other portions.

<Endoscope flexible tube>

The endoscope flexible tube of the present invention includes the sheet for an endoscope flexible tube of the present invention, as an outer skin. Since the sheet for an endoscope flexible tube of the present invention has a high heating residual stress, the endoscope flexible tube of the present invention is unlikely to have a bending habit because plastic deformation is difficult to occur even at the time of autoclave sterilization. Therefore, excellent characteristics are maintained for a long period of time, and satisfactory operability is maintained in a case where the endoscope flexible tube is obtained.

The flexible tube 3a in Fig. 1 has a configuration in which the outer peripheral surface of a flexible tube base material 14 is covered with a resin layer 15 as illustrated in Fig. 2. The sheet for an endoscope flexible tube of the present invention is used as this resin layer 15.

The reference numeral 14a indicates a distal end side (distal end portion 3c side) and the reference numeral 14b represents a proximal end side (main body operation unit 5 side).

The flexible tube base material 14 is obtained by covering a helix tube 11 formed by spirally winding a metal strip 11a on the innermost side, with a cylindrical net body 12 obtained by braiding a metal wire. Each mouthpiece 13 is fitted to both ends thereof. The resin layer 15 is adhered to the flexible tube base material 14 through an adhesive cured material layer 17. The adhesive cured material layer 17 is illustrated as a layer having a uniform thickness for convenience of illustration. However, the adhesive cured material layer 17 may not be necessarily in this form and may be interposed between the resin layer 15 and the flexible tube base material 14 in an amorphous form. The thickness of the adhesive cured material layer 17 may be almost zero so that the resin layer 15 and the flexible tube base material 14 are adhered to each other in the form of being substantially in contact with each other.

The outer surface of the resin layer 15 is coated with a coating layer 16 that contains, for example, fluorine having chemical resistance. Further, in order to clearly illustrate the layer structures, the adhesive cured material layer 17, the resin layer 15, and the coating layer 16 are drawn thicker than the diameter of the flexible tube base material 14.

(Characteristics of sheet for endoscope flexible tube)

Hereinafter, the thickness, hardness, tensile strength, tear strength, and heating residual stress of the sheet for an endoscope flexible tube of the present invention will be described in detail.

[Thickness]

The thickness of the sheet for an endoscope flexible tube of the present invention is not particularly limited, but is preferably in a range of 0.03 mm to 2 mm and more preferably in a range of 0.1 mm to 1 mm.

[Hardness]

In order to improve the operability and the insertion property of the endoscope, the hardness of the sheet is preferably 20 or greater, more preferably 30 or greater, and still more preferably 40 or greater in terms of D hardness. Further, the upper limit thereof is practically 80 or less. The hardness thereof can be measured according to the method described in the section of examples below.

[Tensile strength]

In order to ensure mechanical durability of the sheet for an endoscope flexible tube, the tensile strength of the sheet is preferably 5 MPa or greater, more preferably 10 MPa or greater, and still more preferably 15 MPa or greater. Further, the upper limit thereof is practically 80 MPa or less. The tensile strength thereof can be measured according to the method described in the section of examples below.

[Tear strength]

In order to ensure mechanical toughness of the sheet for an endoscope flexible tube, the tear strength of the sheet is preferably 5 kN/m or greater and more preferably 10 kN/m or greater. Further, the upper limit thereof is practically 15 kN/m or less. The tear strength thereof can be measured according to the method described in the section of examples below.

(Method of producing sheet for endoscope flexible tube)

Hereinafter, an example of a method of producing the sheet for an endoscope flexible tube of the present invention will be described.
It is preferable that a method of producing the sheet for an endoscope flexible tube of the present invention includes a step of mixing a fluorine-containing resin component and a fluorine-containing rubber component, and a step of causing at least parts of at least one of the fluorine-containing resin component or the fluorine-containing rubber component to be polyol-crosslinked, in this order.
Specifically, a fluorine-containing resin component, a fluorine-containing rubber component, a crosslinking agent, and a crosslinking promoter as necessary are mixed, and then heated and kneaded using, for example, a twin screw extruder in a temperature range of 120°C to 250°C for 10 seconds to 10 minutes. Next, the heated mixture is cooled, cut, and pelletized. Subsequently, the obtained pellets are press-molded in a temperature range of 150°C to 300°C for 1 second to 2 hours to promote a crosslinking reaction, thereby obtaining the sheet for an endoscope flexible tube of the present invention.

Examples

Hereinafter, the present invention will be described in more detail based on the following examples. Further, the present invention should not be limitatively interpreted by the following examples.

[Example 1]

80 parts by mass of polyvinylidene fluoride (A-1 of a component (A) listed in Table 1), 15 parts by mass of a vinylidene fluoride-hexafluoropropylene copolymer (B-1 of a component (B) listed in Table 1), 5 parts by mass of a polymer component (C-1 of a component (C) listed in Table 1) having a partial structure formed of a fluorine-containing resin and a partial structure formed of fluorine-containing rubber, 1 part by mass of bisphenol AF, 3 parts by mass of calcium hydroxide, and 1.5 parts by mass of magnesium oxide were put into a 30 mmϕ twin screw extruder whose cylinder temperature was set to 190°C, and the strands were broken after water cooling and then pelletized.
Next, the obtained pellets were press-molded at 250°C for 3 minutes, thereby preparing a sheet for an endoscope flexible tube having a length of 200 mm, a width of 200 mm, and a thickness of 2 mm. Hereinafter, a sheet for an endoscope flexible tube will be simply referred to as a sheet.

[Examples 2 to 10]

A sheet having a length of 200 mm, a width of 200 mm, and a thickness of 2 mm was prepared in the same manner as in Example 1 except that the components (A) to (C) were changed to the compositions listed in Table 1.

[Example 11]

A sheet having a length of 200 mm, a width of 200 mm, and a thickness of 2 mm was prepared in the same manner as in Example 1 except that 2 parts by mass of bisphenol AF, 5 parts by mass of calcium hydroxide, and 2.5 parts by mass of magnesium oxide were used.

[Example 12]

A sheet having a length of 200 mm, a width of 200 mm, and a thickness of 2 mm was prepared in the same manner as in Example 7 except that 2 parts by mass of bisphenol AF, 5 parts by mass of calcium hydroxide, and 2.5 parts by mass of magnesium oxide were used.

[Comparative Example 1]

100 parts by mass of polyvinylidene fluoride (A-1 of a component (A) listed in Table 1) was put into a 30 mmϕ twin screw extruder whose cylinder temperature was set to 190°C, and the strands were broken after water cooling and then pelletized.
Next, the obtained pellets were press-molded at 250°C for 3 minutes, thereby preparing a sheet for an endoscope flexible tube having a length of 200 mm, a width of 200 mm, and a thickness of 2 mm.

[Comparative Examples 2 and 3]

A sheet having a length of 200 mm, a width of 200 mm, and a thickness of 2 mm was prepared in the same manner as in Example 1 except that the components (A) to (C) were changed to the compositions listed in Table 1.

[Comparative Example 4]

A sheet having a length of 200 mm, a width of 200 mm, and a thickness of 2 mm was prepared in the same manner as in Example 1 except that 4 parts by mass of triallyl cyanurate was used as a crosslinking agent and 1.5 parts by mass of 2,5-dimethyl-2,5-di(t-butylperoxy)hexane ("PERHEXA 25B", manufactured by NOF CORPORATION) was used as an organic peroxide, without using bisphenol AF, calcium hydroxide, and magnesium oxide.

[Comparative Example 5]

A sheet having a length of 200 mm, a width of 200 mm, and a thickness of 2 mm was prepared in the same manner as in Example 7 except that 4 parts by mass of triallyl cyanurate was used as a crosslinking agent and 1.5 parts by mass of 2,5-dimethyl-2,5-di(t-butylperoxy)hexane ("PERHEXA 25B", manufactured by NOF CORPORATION) was used as an organic peroxide, without using bisphenol AF, calcium hydroxide, and magnesium oxide.

[Comparative Example 6]

A sheet having a length of 200 mm, a width of 200 mm, and a thickness of 2 mm was prepared in the same manner as in Example 1 except that a crosslinking agent was not used.

[Comparative Example 7]

A sheet having a length of 200 mm, a width of 200 mm, and a thickness of 2 mm was prepared in the same manner as in Example 7 except that a crosslinking agent was not used.
The hardness, tensile strength, tear strength, and heating residual stress of each of the obtained sheets were measured. Hereinafter, the measuring (testing) methods will be described. The results thereof are collectively listed in Table 1.

[Hardness]

The surface hardness of each obtained sheet was measured at 23° using a rubber hardness meter (trade name: RH-201A, manufactured by EXCEL Co., Ltd.) in conformity with the method of JIS K6253 (2006).

[Tensile strength]

No. 2 dumbbell test pieces were prepared from each obtained sheet using a punching mold, and the tensile strength (tensile breaking strength) thereof was measured at 23°C in conformity with the method of JIS K6251 (2004).

[Tear strength]

Angle type test pieces were prepared from each obtained sheet using a punching mold, and the tear strength thereof was measured at 23°C in conformity with the method of JIS K6252 (2007).

[Heating residual stress (heat resistance evaluation)]

The obtained sheets were cut to have test pieces with a size of 80 mm × 10 mm. Stress measurement was performed on each test piece in a high-temperature furnace using a tensile tester (trade name: UCT-30T, manufactured by Orientec Co., Ltd.) having a high-temperature furnace whose temperature was set to 130°C. The test piece was gripped where the distance between chucks was 50 mm, the test piece was rapidly stretched such that the distance between chucks was set to 60 mm (elongation of 20%), and the stress measurement was started. The test piece was held in the stretched state for 15 minutes, and the heating residual stress was calculated based on the following equation. The acceptable level of the heating residual stress is 70% or greater.

[0057] Heating residual stress (%) = stress (N) after test piece was held for 15 minutes/stress (N) after 0 minutes immediately after tensile test × 100

[Repetitive sterilization treatment test in autoclave]

Each of the obtained sheets was prepared to have a size of 100 mm × 100 mm, wound so that the radius thereof was set to 20 mm, and then closed by a clip so as not to open. The resulting sheet was put into an autoclave ("SERVO CLAVE" TW-25AL (trade name), manufactured by TOHO SEISAKUSHO CO., LTD.) in this state, a heated steam treatment carried out at 134°C for 20 minutes was set as one cycle, and 100 cycles of treatments were repetitively performed. The sheet was taken out, the clip was removed, and the evaluation was performed based on the following evaluation standard. The acceptable level is C or higher.

(Evaluation standard)

A: A change in color and/or shape of the sheet was not found before and after the sterilization treatment test and the sheet did not have a bending habit.
B: A change in color and/or shape of the sheet was not found before and after the sterilization treatment test and the sheet had a bending habit, but was easily returned to a flat state by hand.
C: A change in color and/or shape of the sheet was not found before and after the sterilization treatment test and the sheet had a bending habit, but was returned to a flat state by hand in a case where the sheet was strongly pressed and extended by hand.
D: A change in color and/or shape of the sheet was not found before and after the sterilization treatment test, and the sheet repelled hand at the time of being pressed and extended by hand and was not returned to a flat state.
E: A change in color and/or shape of the sheet was found (burning, shrinkage, thermal deformation, or the like was observed).

[Table 1]

| Composition of mixture | | | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| | Component (A) | Type | A-1 | A-2 | A-3 | A-4 | A-5 | A-1 | A-1 | A-1 | A-1 | A-1 |
| | | Content [% by mass] | 80 | 80 | 80 | 80 | 80 | 80 | 80 | 80 | 60 | 40 |
| | Component (B) | Type | B-1 | B-1 | B-1 | B-1 | B-1 | B-2 | B-1 | B-1 | B-1 | B-1 |
| | | Content [% by mass] | 15 | 15 | 15 | 15 | 15 | 15 | 20 | 10 | 30 | 40 |
| | Component (C) | Type | C-1 | C-1 | C-1 | C-1 | C-1 | C-1 | - | C-1 | C-1 | C-1 |
| | | Content [% by mass] | 5 | 5 | 5 | 5 | 5 | 5 | - | 10 | 10 | 20 |
| Crosslinking method | | | Polyol | Polyol | Polyol | Polyol | Polyol | Polyol | Polyol | Polyol | Polyol | Polyol |
| Evaluation | Hardness | [-] | 63D | 50D | 54D | 51D | 52D | 61D | 60D | 64D | 45D | 36D |
| | Tensile strength | [MPa] | 21 | 17 | 18 | 15 | 18 | 20 | 11 | 19 | 14 | 12 |
| | Tear strength | [kN/m] | 12 | 11 | 11 | 12 | 10 | 12 | 8 | 12 | 11 | 10 |
| | Heating residual stress | [%] | 82 | 80 | 81 | 80 | 78 | 81 | 74 | 80 | 84 | 87 |
| | Sterilization treatment test | - | A | B | A | B | B | A | C | B | A | A |

| | | | Example 11 | Example 12 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|
| Composition of mixture | Component (A) | Type | A-1 | A-1 | A-1 | - | A-1 | A-1 | A-1 | A-1 | A-1 |
| | | Content [% by mass] | 80 | 80 | 100 | - | 80 | 80 | 80 | 80 | 80 |
| | Component (B) | Type | B-1 | B-1 | - | B-1 | - | B-1 | B-1 | B-1 | B-1 |
| | | Content [% by mass] | 15 | 20 | - | 100 | - | 15 | 20 | 15 | 20 |
| | Component (C) | Type | C-1 | - | - | - | C-1 | C-1 | - | C-1 | - |
| | | Content [% by mass] | 5 | - | - | - | 20 | 5 | - | 5 | - |
| | Crosslinking method | | Polyol | Polyol | - | Polyol | Polyol | Peroxide | Peroxide | - | - |
| Evaluation | Hardness | [-] | 65D | 63D | 76D | 26D(73A) | 65D | 60D | 56D | 55D | 51D |
| | Tensile strength | [MPa] | 24 | 14 | 45 | 6 | 23 | 16 | 9 | 14 | 7 |
| | Tear strength | [kN/m] | 11 | 8 | 13 | 3 | 12 | 13 | 10 | 8 | 5 |
| | Heating residual stress | [%] | 84 | 76 | 62 | 89 | 68 | 67 | 62 | 61 | 59 |
| | Sterilization treatment test | - | A | C | D | A | D | D | D | D | D |

<Notes in table>

[Fluorine-containing resin component (component (A))]

(A-1) polyvinylidene fluoride (bending elastic modulus of 1500 MPa, melt flow rate of 10 g/10 min): manufactured by Arkema S.A., "KYNAR (registered trademark) 460"
(A-2) ethylene-tetrafluoroethylene copolymer (bending elastic modulus of 780 MPa, melt flow rate of 30 g/10 min): manufactured by DAIKIN INDUSTRIES, Ltd., "NEOFLON ETFE (registered trademark) EP-610"
(A-3) ethylene-tetrafluoroethylene-hexafluoropropylene terpolymer (bending elastic modulus of 1000 MPa, melt flow rate of 25 g/10 min): manufactured by DAIKIN INDUSTRIES, Ltd., "NEOFLON EFEP (registered trademark) RP-5000"
(A-4) tetrafluoroethylene-hexafluoropropylene copolymer (bending elastic modulus of 500 MPa, melt flow rate of 24 g/10 min): manufactured by DAIKIN INDUSTRIES, Ltd., "NEOFLON FEP (registered trademark) NP-101"
(A-5) modified perfluoroalkoxy-based resin (bending elastic modulus of 700 MPa, melt flow rate of 10 g/10 min): manufactured by DAIKIN INDUSTRIES, Ltd., "NEOFLON CPT (registered trademark) LP-1000"

[Fluorine-containing rubber component (component (B))]

(B-1) binary fluororubber (vinylidenefluoride-hexafluoropropylene copolymer, bending elastic modulus of 1 MPa, mass average molecular weight of 310000): manufactured by DAIKIN INDUSTRIES, Ltd., "DAI-EL (registered trademark) G-701"
(B-2) ternary fluororubber (vinylidenefluoride-hexafluoropropylene-tetrafluoroethylene terpolymer, bending elastic modulus of 1 MPa, mass average molecular weight of 280000): manufactured by DAIKIN INDUSTRIES, Ltd., "DAI-EL G-603"

[(Polymer component (component (C)) having partial structure formed of fluorine-containing resin and partial structure formed of fluorine-containing rubber]

(C-1) copolymer (vinylidene fluoride/hexafluoropropylene/ethylene/tetrafluoroethylene block copolymer, bending elastic modulus of 10 MPa, melt flow rate of 22 g/10 min): manufactured by DAIKIN INDUSTRIES, LTD., "DIEL THERMOPLASTIC T-530"
(partial structure formed of fluorine-containing resin: tetrafluoroethylene/ethylene/vinylidene fluoride
partial structure formed of fluorine-containing rubber: vinylidene fluoride/hexafluoropropylene/tetrafluoroethylene) (both trade names)

(Method of measuring bending elastic modulus)

No. 1 dumbbel test pieces were punched from the sheets obtained in the examples and comparative examples, and the bending elastic modulus at 23°C of each test piece was measured using a tensilon universal tester (manufactured by A&D Company, Ltd., RTF-2410 type) in conformity with JIS K 7171:2008.

(Method of measuring melt flow rate)

The melt flow rate was measured under a cylinder temperature condition of 297°C at a load of 49 N using a melt indexer (manufactured by Toyo Seiki Seisaku-sho, Ltd., F-B01 type) in conformity with ASTM D3159.

Based on the results listed in Table 1, the following was found.

Based on the results in Comparative Example 1, the sheet prepared from only the component (A) was not acceptable in terms of the heating residual stress and the sterilization treatment test. The tear strength of the sheet of Comparative Example 2 was significantly low because the sheet was prepared from only the component (B) even though polyol-crosslinking occurred in the sheet. Further, the sheet of Comparative Example 3 was not acceptable in terms of the heating residual stress and the sterilization treatment test because the sheet did not contain the component (B) even through polyol-crosslinking occurred in the sheet.

On the contrary, it was found that the sheets of Examples 1 to 12 had excellent hardness, tensile strength, and tear strength and high heating residual stress (heat resistance) and were unlikely to have a bending habit even in a case where autoclave sterilization was repeatedly performed.

Based on the comparison between the results of Example 1 and the results of Comparative Example 4, even the sheets containing the components (A) to (C) had degraded heat resistance and were not acceptable in terms of the sterilization treatment test in a case where crosslinking other than polyol-crosslinking occurred. Based on the comparison between the results of Example 7 and the results of Comparative Example 5, even the sheets containing the components (A) and (B) had degraded heat resistance and were not acceptable in terms of the sterilization treatment test in a case where crosslinking other than polyol-crosslinking occurred.

Further, based on the comparison between the results of Example 1 and the results of Comparative Example 6, even the sheets containing the components (A) to (C) had degraded heat resistance and were not acceptable in terms of the sterilization treatment test in a case where polyol-crosslinking did not occur. Based on the comparison between the results of Example 7 and the results of Comparative Example 7, even the sheets containing the components (A) and (B) had degraded heat resistance and were not acceptable in terms of the sterilization treatment test in a case where polyol-crosslinking did not occur.

[Chemical resistance]

A chemical resistance test was performed on the sheets of Examples 1 to 12 under the following conditions, and all sheets were acceptable. As the result, it was confirmed that the sheets had excellent chemical resistance.

1) Resistance to ethylene oxide gas (ethylene oxide gas:carbon dioxide = 20:80)

A treatment which was performed by setting the temperature to 55°C, the humidity to 50%, the gas concentration to 450 mg/L, and the sterilization time to 5 hours was set as one cycle, and 100 cycles of treatments were repetitively performed.

2) Resistance to glutaraldehyde aqueous solution

The sheet was immersed in a 0.5 mass% glutaraldehyde-based disinfection solution at 60°C for 3 weeks.

3) Resistance to peracetic acid aqueous solution

The sheet was immersed in a 0.8 mass% peracetic acid-based disinfection solution at 55°C for 3 weeks.

The present invention has been described based on the embodiments, but is not limited to any detailed description unless otherwise specified and should be interpreted broadly without departing from the spirit and the scope of the invention described in the appended claims.

This application claims priority based on JP2016-189760 filed on September 28, 2016 in Japan, the content of which is incorporated herein by reference.

Explanation of References

2:      electronic endoscope (endoscope)
3:      insertion portion
3a:     flexible tube
3b:     angle portion
3c:     distal end portion
5:      main body operation unit
6:      universal cord
11:     helix tube
11a:    metal strip
12:     cylindrical net body
13:     mouthpiece
14:     flexible tube base material
14a:    distal end side
14b:    proximal end side

15:     resin layer
16:     coating layer
17:     adhesive cured material layer

**Claims**

1. A sheet for an endoscope flexible tube which is formed of a composite material that contains a fluorine-containing resin component and a fluorine-containing rubber component and is obtained by polyol-crosslinking of at least parts of the fluorine-containing resin component and the fluorine-containing rubber component.

2. The sheet for an endoscope flexible tube according to claim 1,
   wherein the fluorine-containing resin component contains at least one fluorine-containing resin component selected from polyvinylidene fluoride, an ethylene-tetrafluoroethylene copolymer, an ethylene-tetrafluoroethylene-hexafluoropropylene terpolymer, a tetrafluoroethylene-hexafluoropropylene copolymer, polychlorotrifluoroethylene, or a modified perfluoroalkoxy resin.

3. The sheet for an endoscope flexible tube according to claim 1 or 2,
   wherein the fluorine-containing rubber component contains at least one fluorine-containing rubber component selected from a vinylidene fluoride-hexafluoropropylene copolymer, a vinylidene fluoride-hexafluoropropylene-tetrafluoroethylene terpolymer, tetrafluoroethylene-propylene rubber, a vinylidene fluoride-pentafluoropropylene copolymer, a vinylidene fluoride-chlorotrifluoropropylene copolymer, or tetrafluoroethylene-perfluoromethyl vinyl ether rubber.

4. The sheet for an endoscope flexible tube according to any one of claims 1 to 3,
   wherein the composite material contains a copolymer component having a partial structure formed of one fluorine-containing resin component and a partial structure formed of one fluorine-containing rubber component.

5. An endoscope flexible tube comprising:
   the sheet for an endoscope flexible tube according to any one of claims 1 to 4, as an outer skin.

6. An endoscope comprising:
   the endoscope flexible tube according to claim 5.

7. A method of producing the sheet for an endoscope flexible tube according to any one of claims 1 to 4, comprising:

   a step of mixing a fluorine-containing resin component and a fluorine-containing rubber component; and
   a step of causing at least parts of the mixed fluorine-containing resin component and fluorine-containing rubber component to be polyol-crosslinked.

FIG. 1

FIG. 2

| INTERNATIONAL SEARCH REPORT | International application No. |
|---|---|
| | PCT/JP2017/035077 |

**A. CLASSIFICATION OF SUBJECT MATTER**
Int.Cl. A61B1/005(2006.01)i, G02B23/24(2006.01)i

According to International Patent Classification (IPC) or to both national classification and IPC

**B. FIELDS SEARCHED**

Minimum documentation searched (classification system followed by classification symbols)
Int.Cl. A61B1/00-1/32, G02B23/24-23/26

Documentation searched other than minimum documentation to the extent that such documents are included in the fields searched
Published examined utility model applications of Japan      1922-1996
Published unexamined utility model applications of Japan   1971-2017
Registered utility model specifications of Japan            1996-2017
Published registered utility model applications of Japan   1994-2017

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2014-1360 A (OLYMPUS OPTICAL CO.) 09 January 2014, paragraphs [0005]-[0007], [0012], [0013], [0040], [0048], [0060], [0061] (Family: none) | 1-7 |
| A | JP 11-56762 A (OLYMPUS OPTICAL CO., LTD.) 02 March 1999, paragraphs [0017], [0020] (Family: none) | 1-7 |
| A | JP 2001-275936 A (ASAHI OPTICAL CO., LTD.) 09 October 2001, paragraphs [0059]-[0062], [0110] & US 2001/0029317 A1, paragraphs [0061]-[0064], [0113]-[0116] | 1-7 |

☒ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 27 November 2017 | 05 December 2017 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2017/035077

C (Continuation).   DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No.89551/1986(Laid-open No.200301/1987) (OLYMPUS OPTICAL CO., LTD.) 21 December 1987, entire text, all drawings (Family: none) | 1-7 |
| A | JP 2014-208721 A (OLYMPUS OPTICAL CO.) 06 November 2014, paragraphs [0006], [0048]-[0065] (Family: none) | 1-7 |

Form PCT/ISA/210 (continuation of second sheet) (January 2015)

**REFERENCES CITED IN THE DESCRIPTION**

**Patent documents cited in the description**

- JP 2006081663 A
- JP 2016189760 A